# EUROPEAN PATENT SPECIFICATION

(11) **EP 4 013 475 B1**
(45) Date of publication and mention of the grant of the patent: **27.09.2023**
(21) Application number: 21746187.0
(22) Date of filing: 01.07.2021
(51) Int. Cl.: A61M 5/315

(54) **SEMI-AUTOMATICALLY CONTROLLABLE SYRINGES**
HALBAUTOMATISCH KONTROLLIERBARE SPRITZEN
SERINGUES A COMMANDE SEMI-AUTOMATIQUE

(30) Priority: 01.07.2020 IN 202041028073
(43) Date of publication of application: 22.06.2022
(73) Proprietor: Payeli, Sravan Kumar, Karnataka, Bangalore 560064 (IN)
(72) Inventor: MOCHARLA, Pavani Srividya, Karnataka Bangalore 560064 (IN); PAYELI, Sravan Kumar, Karnataka Bangalore 560064 (IN)
(74) Representative: Regimbeau
(86) International application number: PCT/IN2021/050644
(87) International publication number: WO 2022/003728

(56) References cited:
- US-A- 2 823 674
- US-A- 2 952 255
- US-A- 4 546 859

## Description

### TECHNICAL FIELD

The present subject matter relates, in general, to syringes for injecting fluid into a target body or to create positive pressure, and extracting or collecting samples from, a target body or to create aspiration pressure, and particularly relates to semi-automatically controllable syringes for said purposes.

### BACKGROUND

Various applications, such as medical applications, laboratory applications, and common workshop applications utilize syringes for performing different procedures, such injection of doses of a fluid, such as a drug, into a target body, extraction of samples and biofluids, such as blood, measurement and transferring of chemicals, and injection of substances, such as, glue and lubricants, onto working surfaces. The working of the syringe requires application of force for performing operations, including, injection and extraction, that creates a required positive and negative pressure, respectively, which can be further applied in biological applications, such as for holding a biological membrane or body in place to perform a required procedure, or for puncturing a biological membrane or body.

US 4546859 describes a syringe according to the preamble of claim 1, while US 2823674 and US 2952255 illustrate the background of the invention.

### BRIEF DESCRIPTION OF DRAWINGS

The features, aspects, and advantages of the subject matter will be better understood with regard to the following description and accompanying figures. The use of the same reference number in different figures indicates similar or identical features and components.
FIGS. 1A to 1E illustrate semi-automatically controllable syringes, in accordance with an implementation of the present subject matter;
FIG. 2 illustrates a semi-automatically controllable syringe, in accordance with an implementation of the present subject matter;
FIG. 3 illustrates a semi-automatically controllable syringe, in accordance with an implementation of the present subject matter;
FIG. 4 illustrates a semi-automatically controllable syringe, in accordance with an implementation of the present subject matter;
FIGS. 5A and 5B illustrate a pneumatically actuated semi-automatically controllable syringe 500, in accordance with an implementation of the present subject matter;
FIG. 6 illustrates a pneumatically actuated semi-automatically controllable syringe, in accordance with an implementation of the present subject matter;
FIG. 7 illustrates a syringe assembly, in accordance with an implementation of the present subject matter;
FIG. 8A illustrates a longitudinal semi-automatically controllable syringe, in accordance with an implementation of the present subject matter;
FIG. 8B illustrates an injection assembly, in accordance with an implementation of the present subject matter; and
FIG. 9 illustrates a graphical representation of the performance comparison between a conventional syringe and an exemplary semi-automatically controllable syringe with respect to data points representing pressure values measured at an injection needle side for an intracytoplasmic sperm injection process.

### DETAILED DESCRIPTION

A conventional syringe includes a barrel, a plunger, an adapter, and a needle. The needle is attached to a first barrel end via the adapter, and the plunger is partially placed inside the barrel from a second barrel end. A cylindrical barrel chamber is enclosed by the first barrel end and a first plunger end, which is disposed inside the barrel. The barrel chamber is to contain a fluid. The plunger includes a plunger flange disposed at a second plunger end, which is axially opposite to the first plunger end. The plunger flange is positioned external to the barrel. A barrel flange is disposed at a second barrel end located distal to the adapter. The plunger flange and the barrel flange are to allow for application of a pull force or a push force by placing at least one finger each at respective inner sides of the plunger flange and the barrel flange, the inner side of the plunger flange facing towards the inner side of the barrel flange. The application of the pull force allows for a movement of the first plunger end away from the needle along an axis of the cylindrical chamber, which reduces a pressure inside the barrel chamber. The reduction in the pressure inside the barrel chamber exerts an inward force, towards the barrel chamber. In case an open end of the needle is brought in contact with a fluid, the fluid is sucked into the barrel chamber via the needle, with the effect of the coupling of the needle with the barrel chamber. The suction of the fluid inside the barrel chamber results in the extraction of the fluid from a source of the fluid.

Alternatively, in order to perform an injection operation with the syringe, at least one finger each is placed on respective outer sides of the plunger flange and the barrel flange. The outer side of the plunger flange and the outer side of the barrel flange face in directions opposite to each other. A push force is applied on the plunger flange, which results in a movement of the plunger towards the needle. The movement of the plunger towards the needle increases the pressure inside the barrel chamber, and, in turn, forces a fluid present inside the barrel chamber to be ejected out of the barrel chamber via the opening of the needle. In order to inject the fluid into a target body, the needle opening is pierced inside an outer surface of the target body.

However, in the conventional syringe, in order to perform the extraction and injection operations with the syringe, a user is required to use at least two fingers for exerting the pull and push forces, while rest of the fingers are used to hold the syringe in place. This may result in an unstable positioning of the syringe while in operation, and in turn resulting in an unstable syringe operation. In addition, the user may experience inconvenience in operating the syringe, as it requires placement of two fingers on the plunger flange and the barrel flange for application of pressure.

Further, in applications requiring a specific target pressure value during the operation of the syringe, use of the conventional syringe may incur challenges as the pressure applied by the same user in different instances may not exactly match with the target pressure value. Similarly, a mismatch may occur in values of pressure which is being applied by two different users. In applications requiring extraction and injection of precise amount of fluid, this mismatch in two different pressure values may result in an undesirable outcome, with respect to the extraction and injection processes. In addition, in order to achieve the target pressure value, the user may take more time to perform the syringe operation. Therefore, there is a need for a syringe which can provide a control over the applied pressure so as to avoid mismatch in the values of pressure applied relative to a target pressure value across different users. Further, there is a requirement of a syringe which may improve the convenience of the user in holding and applying pressure in order to perform the syringe operation.

Another conventional syringe, such as a rotary syringe may be used for performing different procedure, for example, in a medical application. For example, the rotary syringe is used in the application of in vitro fertilization, such as in intracytoplasmic sperm injection. For performing intracytoplasmic sperm injection, the rotary syringe is utilized for collecting a sperm, puncturing of a layer (oolemma) of an oocyte, and for injecting the collected sperm into an oocyte. Further, similar rotary syringe may be used for holding the oocyte in place for performing the sperm injection. This application requires a high degree of precision in order to efficiently perform the required operation. In general, a conventional rotary syringe includes a rotatable cylindrical actuator which is disposed on a base. The actuator includes a body with threaded inner surface and a piston having threads which complement with the threads of the body. The threads of the piston may engage with the threads of the body. Further, the body may be connected with a thin tube connected to a needle unit. In order to actuate the syringe, the actuator may be rotated clockwise to create a negative pressure in the syringe. The negative pressure may be created to collect one or more sperms into the syringe. Further, a sudden burst of aspiration or a negative pressure may also be created for puncturing the oolemma.

Alternatively, for injection of the collected sperm in the oocyte, the actuator may be rotated anti-clockwise, which creates a positive pressure in the syringe. The positive pressure may result in the ejection of the sperm towards an opening of the needle. The needle may be placed in contact with the target region of the oocyte, and further positive pressure may be applied for injecting the sperm into the oocyte. However, application of an undesired amount of negative pressure may result in an undesired outcome, such as undesired collection of fluid having the sperms or undesired collection of fluid from cytoplasm of the oocyte upon puncturing of the oolemma. The undesired positive pressure may also result in damaging of the oocyte during the process of sperm injection.

Similarly, a needle of a separate conventional rotary syringe may be placed in proximity of the target oocyte, and the negative pressure may be created in the separate rotary syringe in order to hold the oocyte in the desired position to perform sperm injection. However, the holding negative pressure applied for holding the oocyte, may be precisely applied and controlled in order to prevent damaging of the oocyte during the sperm injection process.

It may be noted that the amount of negative and positive pressures applied by different users or by the same user at different instances may vary with a high degree, which may introduce undesired subjectivity to the process of oocyte holding and oolemma puncturing, during sperm collection and injection. Further, in order to reduce the degree of subjectivity, a user may be required to apply specific, precise pressures on to the actuator during the operation of the syringe. This may result in an increase in the overall time need to perform the operation.

Therefore, there is requirement of syringes which may eliminate the subjectivity involved in the handling of the syringes to perform medical procedure that require higher degree of precision and control and reduce the amount of time required to perform the required process.

The present subject matter describes semi-automatically controllable syringes with improved degree of control during the operation, higher convenience of use, and reduced amount of time required for operation.

In accordance with an example of the present subject matter, a semi-automatically controllable syringe includes a barrel, a plunger, an adapter, a needle, a biasing member, an actuating member, and a movement control mechanism. The plunger is moved to a direction towards the needle by applying a push force on the actuating member, forming a positive pressure inside the barrel. The biasing member is coupled with the plunger, and upon the movement of the plunger in the direction towards the needle, the biasing member attains a biased state. Upon release of the push pressure, the biasing member applies a restoring force on the plunger away from the needle and returns to an original position. The plunger also returns to the original position in conjunction with the movement of the biasing element.

Alternatively, the plunger is moved to a direction away from the needle by applying a pull force on the actuating member, forming a negative pressure inside the barrel. Upon the movement of the plunger in the direction away from the needle, the biasing member attains a biased state. Upon release of the pull pressure, the biasing member applies a restoring force on the plunger towards the needle and returns to an original position. The plunger returns to the original position in conjunction with the movement of the biasing element.

Since the plunger is returned to its original position by the restoring force of the biasing member, no external force is required for the same. In addition, the user is not required to change his grip of the syringe for returning the plunger to its original position, which facilitates in maintaining the syringe in a stable position during the operation of the syringe. In addition, the user is able to perform the syringe operation in a comfortable and convenient manner.

Further, the movement control mechanism of the syringe includes a pin and a set of pin-receiving members. The pin is insertable in any of the pin-receiving members, from the set of pin-receiving members. The pin, upon insertion into any of the pin-receiving members provides a variable limit for the movement of the plunger within the barrel. For example, the movement control mechanism may limit the movement of the plunger in a direction in which the plunger is actuated by the user. Such variable limiting of the movement of the plunger defines and limits a range of pressure to be applied by the user for the operation of the syringe. The movement control mechanism of the syringe facilitates in substantially reducing the efforts of the user for applying and maintaining a precise force during the push or pull operation of the plunger, thereby reducing the overall time taken by the user to the perform the syringe operation. The movement control mechanism also helps in applying and maintaining substantially uniform force during the push or pull operation of the plunger, which eliminates subjectivity in the syringe operation because of different users or use at different instances and prevents undesirable outcomes with respect to the processes carried out by the syringe.

Accordingly, the approaches of the present subject matter provide semi-automatically controllable syringes, which ensures a convenient and efficient controlling of syringe while improving the stability of syringe operation. Further, controlling the movement of the plunger, as implemented by the present subject matter, allows for eliminating introduction of subjectivity in the syringe operation and reducing an overall time required for the syringe operation.

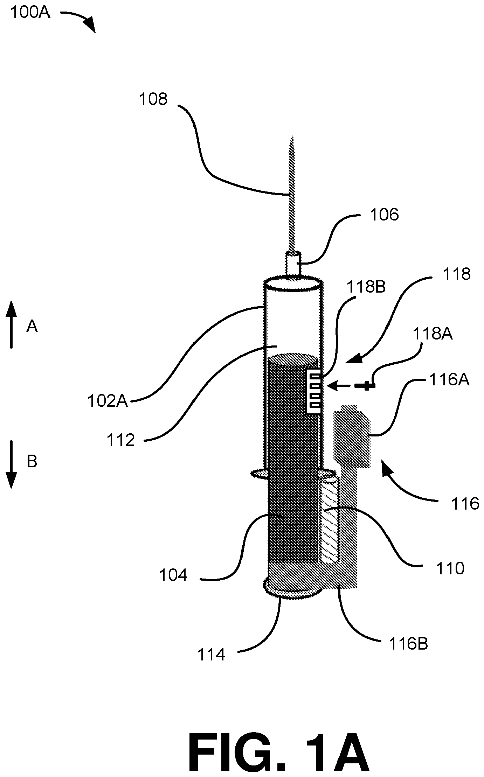

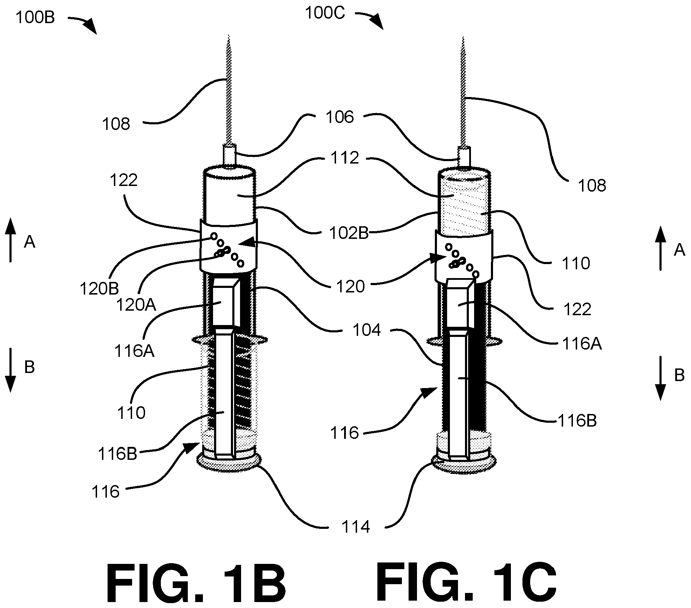

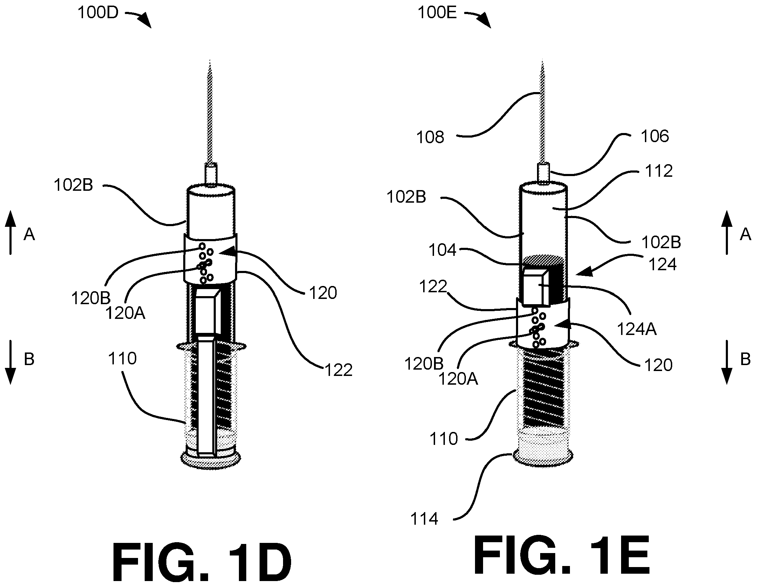

These and other advantages of the present subject matter would be described in a greater detail in conjunction with FIGS. 1-9 in the following description. The manner in which the devices of present subject matter are implemented shall be explained in detail with respect to FIGS. 1-9. It should be noted that the description merely illustrates the principles of the present subject matter. It will thus be appreciated that those skilled in the art will be able to devise various arrangements that, although not explicitly described herein, embody the principles of the present subject matter and are included within its scope. Furthermore, all examples recited herein are intended only to aid the reader in understanding the principles of the present subject matter. Moreover, all statements herein reciting principles, aspects and implementations of the present subject matter, as well as specific examples thereof, are intended to encompass equivalents thereof.

FIGS. 1A to 1E illustrate semi-automatically controllable syringes 100A to 100E, in accordance with an implementation of the present subject matter. The semi-automatically controllable syringe 100A to 100E is also referred hereinafter as the syringe 100A to 100E for the sake of brevity. The semi-automatically controllable syringes 100A includes a barrel 102A, a plunger 104, an adapter 106, a needle 108, a biasing member 110, and a chamber 112, a cap 114, an actuating member 116, and a movement control mechanism 118. The barrel 102A may include an orifice and an internal cavity. The plunger 104 may be partially arranged inside the barrel 102A. The plunger 104 may be coupled with the biasing member 110. The plunger 104 may be movably arranged inside the barrel 102A in a slidable manner. The adapter 106 is integrated at a first end of the barrel 102A. The barrel 102A includes an opening at a second end which is located opposite to the first end. The plunger 104 is inserted into the barrel 102A from the opening of the second end. In an example, the plunger 104 is at least partially disposed in the internal cavity of the barrel 102A and is slidable in a first direction and a second direction opposite to the first direction in the internal cavity.

The plunger 104 includes an actuating end and an operating end. In the partially arranged state, the operating end of the plunger 104 is disposed inside the barrel 102A, and the actuating end of the plunger 104, which is to receive a force or actuating the plunger 104, is away from the operating end.

In an example, the operating end of the plunger 104 includes a sealing element (not shown in FIG. 1A) for creating an air-tight seal around an inner surface of the barrel 102A. The sealing element includes a rubber gasket. The cap 114 is attached to the actuating end of the plunger 104, in order to create a flange-type structure at the actuating end. In an example, the cap 114 is attached to the actuating end through a threaded connection. In another example, the cap 114 is snap-fitted onto the actuating end. The second end of the barrel is disposed with a flange. The biasing member 110 is disposed between the flange-type structure disposed on the plunger 104 and the flange of the barrel 102A. An end of the biasing member 110 is fitted to the cap 114 and the cap 114 locks the biasing member 110 between the flange-type structure disposed on the plunger 104 and the flange of the barrel 102A. In an example, the biasing member 110 is to bias upon movement of the plunger in the first direction and is to apply a restoring force on the plunger 104 towards the second direction. In an example, the biasing member is a mechanical spring or a wire spring.

The needle 108 is attached to the adapter 106. The first end of the barrel 102A and the operating end of the plunger 104 defines the chamber 112.

In order to carry out an operation, for example, collection of sample in the syringe 100A, the plunger 104 is moved in direction A by applying a push force on the actuating end of the plunger 104 towards the needle 108. Simultaneously, the movement of the plunger 104 in the direction A results in biasing of the biasing member 110. Further, an opening end of the needle 108 is positioned in contact with a target fluid. The biasing member 110 remains in the biased state with the effect of the applied push force.

For initiation of sample collection, the push force is removed from the plunger 104, which results in movement of the biasing member 110 back to the unbiased state. As a result of coupling of the biasing member 110 with the plunger 104, the plunger 104 is moved away from the needle 108, in conjunction to the unbiasing of the biasing member 110. The movement of the plunger 104 away from the needle 108 results in creation of negative pressure inside the chamber 112. This negative pressure causes a movement of the fluid, in contact with the opening end, in direction B towards the chamber 112 via the needle 108. The collection of sample is completed when the biasing member 110 and the plunger 104, in conjunction to the movement of the biasing member 110, reach to an original position, which is the unbiased state of the biasing member 110.

As shown in FIG. 1A, the actuating member 116 is coupled with the plunger 104. For example, the actuating end of the plunger 104 is attached to the actuating member 116. The user may grip the syringe 100A with his fingers and place his thumb on the actuating member 116 to slide the actuating member 116 in the direction A. The actuating member 116, upon application of an external force thereon by the user, causes actuation of the plunger 104 in the direction A. The actuating member 116 includes a button 116A and a connector 116B. The button 116A is coupled to the actuating end of the plunger 104 via the connector 116B. The biasing member 110 is disposed between the second end of the barrel 102A and a section of the connector 116B. The actuating member 116, upon application of an actuating force thereon, is to cause a movement of the plunger 104 in the first direction. In an example, the actuating member 116 is a linear actuating member.

The actuating member 116 as shown in Fig. 1A is a mechanical actuating member. However, in another example, the actuating member may be one of a hydraulic actuating member, a pneumatic actuating member, and an electrical actuating member.

The movement control mechanism 118 of the syringe 100A includes a pin 118A and a set of pin-receiving members 118B. The pin 118A is insertable in any of the pin-receiving members 118B, from the set of pin-receiving members 118B, to provide a variable limit for the movement of the plunger 104 within the barrel 102A, in the first direction. In the semi-automatically controllable syringe 100A, the set of pin-receiving members 118B are on an outer surface of the barrel 102A. The alignment of each of the pin-receiving members 118B is such that insertion of the pin 118A in each of the pin-receiving members 118B allows for a different limit to the movement of the plunger 104. The plunger 104 is limited to move only up till that pin-receiving member in which the pin 118A is inserted.

The set of pin-receiving members 118B in the syringe 100A is a set of holes formed on the barrel 102A. The pin 118A is inserted in one of the pin-receiving members 118B. The pin 118A protrudes in the internal cavity of the barrel 102A. Further, the movement of the plunger 104 towards the needle 108, upon application of the external force, causes the shaft of the plunger 104 to abut to the pin 118A in the internal cavity. In an example, the shaft may include a flange (not shown in FIG. 1A) which abuts with the pin 118A to limit the movement of the plunger 104 in direction A.

In another example, the pin 118A, when inserted in one of the pin-receiving members 118B, protrudes out of the barrel 102A and the actuating member 116 abuts with the pin 118A to limit the movement of the actuating member 116 and therefore the plunger 104 in direction A. In an example, the button 116A of the actuating member 116 abuts the pin 118A.

The restriction to the movement of the plunger 104 in the barrel 102A provided by the movement control mechanism 118 allows for a substantially uniform control over the movement of the plunger 104, elimination of the non-uniformity over the actuating force applied onto the plunger 104 by different users or at different instances.

FIG. 1B illustrates an exemplary semi-automatically controllable syringe 100B which includes the plunger 104, the adapter 106, the needle 108, the biasing member 110, and the chamber 112, the cap 114, and the actuating member 116, similar to the semi-automatically controllable syringes 100A. Further, the semi-automatically controllable syringes 100B includes a barrel 102B and a movement control mechanism 120 including a pin 120A and a set of pin-receiving members 120B. The biasing member 110 is disposed between a flange-type structure disposed on the plunger 104 and a flange of the barrel 102B.The movement control mechanism 120 includes a sleeve 122 mountable on the barrel 102B. The set of pin-receiving members 120B are on the sleeve 122. In an example, the sleeve 122 is mounted on the barrel 102B. In an example, the set of pin-receiving members 120B are aligned along a straight line inclined with respect to a longitudinal axis of the semi-automatically controllable syringe 100B. In an example, the set of pin-receiving members 120B is a set of holes to receive the pin 120A. In another example, the set of pin-receiving members 120B is a set of cavities to receive the pin 120A.

The pin 120A is inserted in one of the pin-receiving members 120B. The pin 120A protrudes out from the barrel 102B and abuts with the button 116A upon the movement of the plunger 104 in direction A. Such abutting limits further movement of the actuating member 116 in direction A. Further, due to the coupling of the actuating member 116 with the plunger 104, the movement the plunger 104 is also limited in direction A.

FIG. 1C illustrates an exemplary semi-automatically controllable syringe 100C which includes the barrel 102B, the plunger 104, the adapter 106, the needle 108, the biasing member 110, the chamber 112, the cap 114, the actuating member 116, the movement control mechanism 120, and the sleeve 122, similar to the semi-automatically controllable syringe 100B. The biasing member 110 is disposed between a first end of the barrel 102B which is proximal to the adapter 106 and an operating end of the plunger 104.

In the implementations shown in FIGS. 1A to 1C, in conjunction to the movement of the plunger 104 in direction A, the biasing member 110 attains a compressed biased state from an unbiased state. Further, in conjunction to the movement of the plunger 104 in direction B, the biasing member 110 returns to the unbiased state from the compressed biased state.

The biasing member 110 allows to automate the movement of the plunger 104 back to its original position As the manual pulling back of the plunger 104 is not required, the user may comfortably hold the syringe 100A to 100C by gripping the barrel 102B and conveniently perform the required syringe operation. This improves the overall stability of the syringe during the operation. Further, the time required to perform the collection process is reduced as a latter portion of the collection process, involving creation of the negative pressure, is automated.

FIG. 1D illustrates an exemplary semi-automatically controllable syringe 100D. The functioning of the semi-automatically controllable syringes 100D is similar to the functioning of the semi-automatically controllable syringes 100B. The set of pin-receiving members 120B are aligned on a zig-zag line.

FIG. 1E illustrates an exemplary semi-automatically controllable syringe 100E which includes the barrel 102B, the plunger 104, the adapter 106, the needle 108, the biasing member 110, the chamber 112, and the cap 114, similar to the semi-automatically controllable syringe 100B. The semi-automatically controllable syringe 100E includes a movement control mechanism 120 and a sleeve 122 similar in structure to the movement control mechanism 120 and the sleeve 122 of FIG. 1D. The semi-automatically controllable syringe 100E also includes an actuating member 124 including a button 124A. The actuating member 124 is coupled with the plunger 104. For example, the button 124A of the actuating member 124 is coupled to the plunger 104 through a slot made on the barrel 102B. The sleeve 122 of the semi-automatically controllable syringe 100E, as shown, is mounted on the barrel 102B at a position between the button 124A and an end of the barrel 102B which is opposite to the end at which the adapter 106 in integrated.

The pin 120A is inserted in one of the pin-receiving members 120B. The pin 118A protrudes out from the barrel 102B and abuts with the button 116A upon the movement of the plunger 104 in direction B. Such abutting limits further movement of the actuating member 116 in direction B. Further, due to the coupling of the actuating member 116 with the plunger 104, the movement of the plunger 104 is also limited in direction B.

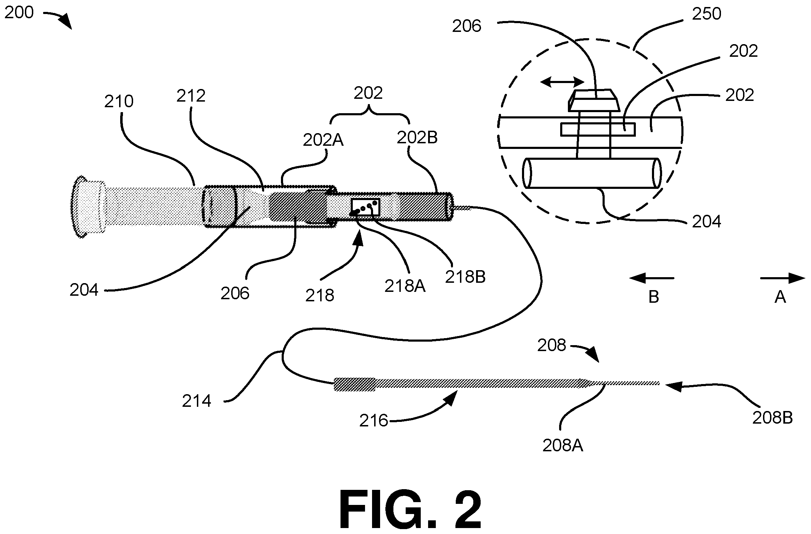

FIG. 2 illustrates a semi-automatically controllable syringe 200, in accordance with an implementation of the present subject matter. The semi-automatically controllable syringe 200 is used in an In-vitro fertilization process, such as an intracytoplasmic sperm injection. The semi-automatically controllable syringe 200, hereinafter also referred to as the syringe 200 for the sake of brevity, may be used for holding an oocyte in place by applying negative pressure. The syringe 200 includes a barrel 202, a plunger 204, an actuating member 206, a needle 208, a biasing member 210, a chamber 212, a tube 214, a needle holder 216, and a movement control mechanism 218. The biasing member 210 and the movement control mechanism 218 are similar to the biasing member 110 and the movement control mechanism 120, respectively. The barrel 202 includes a first section 202A and a second section 202B. The diameter of the first section 202A is greater than the diameter of the second section 202B. The plunger 204 is arranged inside the barrel 202. In an example, the plunger 204 is partially arranged inside the barrel 202. In another example, the plunger 204 is completely arranged inside the barrel 202. The barrel 202 includes a slot 220. The actuating member 206 is disposed on the barrel 202 and coupled with the plunger 204 via the slot 220. In FIG. 2, encircled region 250 shows a side-sectional view of the coupling of the actuating member 206 with the plunger 204 via the slot 220. The slot 220 is long enough so that the actuating member 206 and the plunger 204 can slide in directions A and B without any hinderance. The biasing member 210 is arranged between the plunger 204 and the barrel 202. An operating end of the plunger 204 and a first end of the barrel 202 that is proximal to the adapter defines the chamber 212.

The barrel 202 is connected with the needle 208 via the tube 214. The length of the tube 214 may be in a range of 70-200 cm. An inner diameter of the tube 214 may be 1 mm. The needle 208 is mounted on the needle holder 216 for stably holding the needle 208. The needle 208 includes a hollow shaft 208A with a tip 208B. An inner diameter of the tip 208B may be in a range of 20-50 microns. In an example, the needle 208 is a disposable needle. The needle holder 216 may be mounted on a manipulator controller (not shown) in order to precisely control the positioning of the needle 208.

The plunger 204 is moved towards the tube 214 upon application of a push force on an actuating end of the plunger 204, located opposite to the operating end of the plunger 204, in order to create positive pressure inside the chamber 212. As a result of this movement, the biasing member 210 attains a biased state. In an example, a displacement of the plunger 204 may be in a range of 1-5 cm. The tip 208B is positioned in proximity of an oocyte which is to be injected with a sperm. Upon positioning the tip 208B in proximity to the oocyte, the push pressure is released. Upon release of the push pressure, the biasing member 210 returns to an unbiased state.

With effect of the movement of the biasing member 210 to the unbiased state, the plunger 204 is moved automatically towards an original position which creates a negative pressure inside the chamber 212. As a result, the oocyte is sucked towards the tip 208B. Due to a large size of the oocyte as compared with the tip 208B, the oocyte is held at the tip 208B, and is prevented from getting completely sucked inside the needle 208. The displacement of the plunger 204 exerts a negative pressure in a range of 1-3 kilo-pascal (kPa).

In addition, a range of displacement of the plunger 204 and the biasing member 210 is controlled with the movement control mechanism 218 disposed on the barrel 202, which restricts the movement of the plunger 204 to a required degree. The movement control mechanism 218 includes a pin 218A and a set of pin-receiving members 218B. The pin 218A is inserted in one of the pin-receiving members 218B. The pin 218A protrudes out from the barrel 202 and abuts with the actuating member 206 upon the movement of the actuating member 206 in direction A. Such abutting limits further movement of the actuating member 206 in direction A. Further, due to the coupling of the actuating member 206 with the plunger 204, the movement of the plunger 204 is also limited in direction A. Similarly, by inserting the pin 218A in different pin-receiving members 218B a user may increase or decrease the range in accordance with the required operation. The controlling of the range allows the user to precisely control an amount of negative pressure applied to hold the oocyte at the tip 208B.

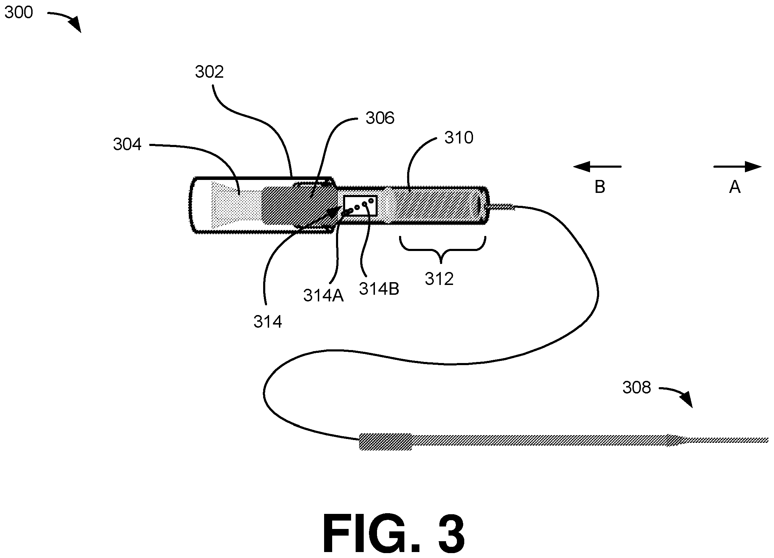

FIG. 3 illustrates a semi-automatically controllable syringe 300, in accordance with an implementation of the present subject matter. The semi-automatically controllable syringe 300, hereinafter also referred to as the syringe 300 for the sake of brevity, may be used in the In-vitro fertilization process, such as an intracytoplasmic sperm injection. The syringe 300 includes a barrel 302, a plunger 304, an actuating member 306, a needle 308, a biasing member 310, a chamber 312, and a movement control mechanism 314. The plunger 304, the actuating member 306, the needle 308, and the movement control mechanism 314 are similar to the plunger 204, the actuating member 206, the needle 208, and movement control mechanism 218, respectively. The barrel 302 is closed from an end which is distal to the end of the barrel 302 connected with the needle 308. The biasing member 310 of the syringe 300 is connected between a first end of the barrel 302, located in proximity to the needle 308, and an operating end of the plunger 304, which defines the chamber 312, similar to the chamber 212 of FIG. 1.

The movement control mechanism 314 controls a range of displacement of the plunger 304 and the biasing member 310 by restricting the movement of the plunger 304 to a required degree. The movement control mechanism 314 includes a pin 314A and a set of pin-receiving members 314B. The pin 314A is inserted in one of the pin-receiving members 314B. The pin 314A protrudes out from the barrel 302 and abuts with the actuating member 306 upon the movement of the actuating member 306 in direction A. Such abutting limits further movement of the actuating member 306 in direction A. Further, due to the coupling of the actuating member 306 with the plunger 304, the movement of the plunger 304 is also limited in direction A. Similarly, by inserting the pin 314A in different pin-receiving members 314B a user may increase or decrease the range in accordance with the required operation. The controlling of the range allows the user to precisely control an amount of negative pressure applied to hold the oocyte at a tip 308A of the needle 308.

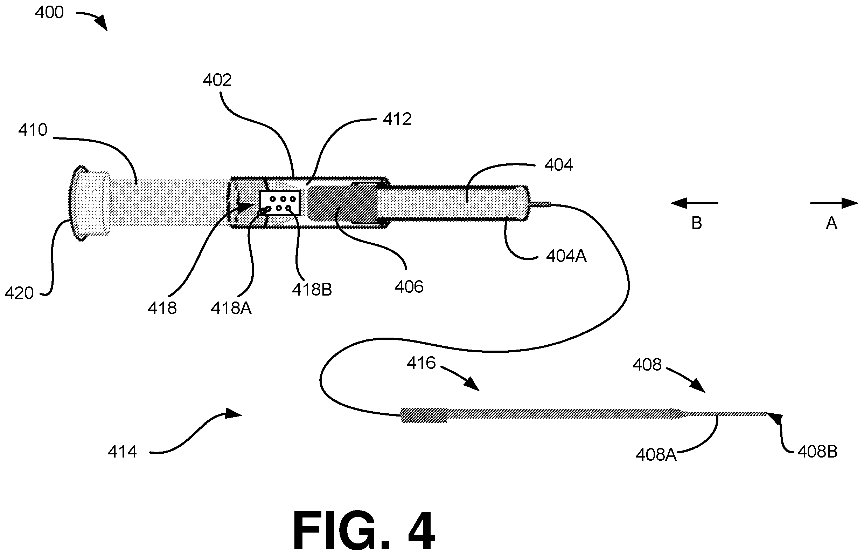

FIG. 4 illustrates a semi-automatically controllable syringe 400, in accordance with an implementation of the present subject matter. The semi-automatically controllable syringe 400, hereinafter also referred to as the syringe 400 for the sake of brevity, may be used for collecting a sperm, and for injecting the collected sperm in a target oocyte in an In-vitro fertilization process, such as an intracytoplasmic sperm injection. The syringe 400 includes a barrel 402, a plunger 404, an actuating member 406, a needle 408, a biasing member 410, a chamber 412, a tube 414, a needle holder 416, and a movement control mechanism 418. A structural arrangement of the features of the syringe 400, may be similar to the structural arrangement of the features of the syringe 200 of FIG. 2. However, structural dimensions of the features of the syringe 400 may be different from the features of the syringe 200 and the semi-automatically controllable syringe 100E. An operating end of the plunger 404, positioned away from the biasing member 410, and a first end of the barrel 402, located away from the biasing member 410, defines the chamber 412. The barrel 402 is connected with the needle 408 via the tube 414. The length of the tube 414 may be in a range of 70-200 cm. An inner diameter of the tube 414 may be 1 mm.

The needle 408 is mounted on the needle holder 416 for stably holding the needle 408. The needle 408 includes a hollow shaft 408A with a tip 408B. An inner diameter of the tip 408B may be in a range of 3-10 microns. In an example, the needle 408 is a disposable needle. The needle holder 416 is mounted on a manipulator controller (not shown) in order to precisely control the positioning of the needle 408. The plunger 404 is moved towards the tube 414 by applying a push force on the plunger 404 in order to create a positive pressure inside the chamber 412. As a result of such movement, the biasing member 410 attains a biased state.

In an example, the operating end of the plunger 404 includes a sealing element 404A for creating an air-tight seal around an inner surface of the barrel 402. The plunger 404 includes an actuating end located opposite to the operating end. The sealing element 404A includes a rubber gasket. A cap 420 is attached to a second end of the barrel 402, where the second end is located opposite to the first end of the barrel 402. The cap 418 creates a flange-type structure at the second end of the barrel 402. In an example, the cap 420 is attached to the second end of the barrel 402 through a threaded connection. In another example, the cap 420 is snap-fitted onto the second end of the barrel 402. The actuating end of the plunger 404 is disposed with a flange. The biasing member 410 is disposed between the flange-type structure disposed on the second end of the barrel 402 and the flange of the actuating end of the plunger 404. An end of the biasing member 410 is fitted to the cap 420 and the cap 420 locks the biasing member 410 between the flange-type structure disposed on the second end of the barrel 402 and the flange of the actuating end of the plunger 404.

The actuating member 406 is used by the user to cause a movement of the plunger 404. In an example, the movement of the plunger 404 may be in a range of 1-5 cm. A movement of the plunger 404 towards the cap 420 exerts a negative pressure in a range of 10-20 kPa.

The movement control mechanism 418 controls a range of displacement of the actuating member 406 by restricting the movement of the actuating member 406 to a required degree. Such controlling also limits a range of displacement of the plunger 404 due to the coupling of the actuating member 406 with the plunger 404. The movement control mechanism 418 includes a pin 418A and a set of pin-receiving members 418B. The pin 418A is inserted in one of the pin-receiving members 418B. The pin 418A protrudes out from the barrel 402 and abuts with the actuating member 406 upon the movement of the actuating member 406 in direction B. Such abutting limits further movement of the actuating member 406 in direction B. Further, due to the coupling of the actuating member 406 with the plunger 404, the movement of the plunger 404 is also limited in direction B. Similarly, by inserting the pin 418A in different pin-receiving members 418B a user may increase or decrease the range in accordance with the required operation. The controlling of the range allows the user to precisely control an amount of pressure required for collecting a sperm and for injecting the collected sperm in a target oocyte in an In-vitro fertilization process.

A mechanical pressure is applied by the needle 408 onto an oocyte (not shown), in which a sperm (not shown), which is collected inside the tube 414, in order for the tip 408B to pierce through an outer layer (zona pellucida) of the oocyte. After puncturing the outer layer, the needle 408 is pushed further towards the oocyte into 30% to 70% of the oocyte diameter. Further, the plunger 404 is applied with a pull force on to the actuating member 406 towards the cap 420 in controllable manner to create a negative pressure or aspiration pressure, which, in turn, actuates the biasing member 410 to attain a biased state in a stretched manner. The negative pressure is utilized to puncture an inner layer of the oocyte (oolemma). After successful puncture of the inner layer, the pull force is released. The release of the push force allows the biasing member 410 to automatically attain an unbiased state from the biased state. The change of the biasing member 410 to the unbiased state allows the movement of the plunger 404 in order to neutralize the applied negative pressure. After, the biasing member 410 is returned to the unbiased state, in addition, a positive pressure is applied to deposit the sperm into the oocyte. In an example, the positive pressure may be in a range of 1-2 kPa. This results in successful deposition of the sperm into oocyte cytoplasm.

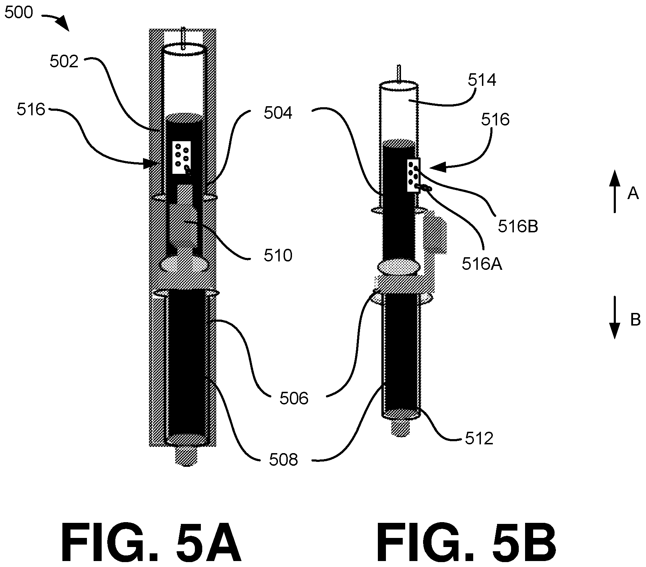

FIGS. 5A and 5B illustrate a pneumatically actuated semi-automatically controllable syringe 500, in accordance with an implementation of the present subject matter. The pneumatically actuated semi-automatically controllable syringe 500 is also referred hereinafter as the pneumatic syringe 500 for the sake of brevity. FIG. 5A illustrates a cross-sectional view of the pneumatic syringe 500 with the outer casing 502. FIG.5B illustrates the pneumatic syringe 500 without the outer casing 502. The pneumatic syringe 500 include an operating barrel 504, an actuating barrel 506, a plunger 508, an actuating member 510, a sealing cap 512, a chamber 514, and a movement control mechanism 516. The movement control mechanism 516 may be similar to the movement control mechanism 218 of FIG. 2. The operating barrel 504 and the actuating barrel 506 is coupled to define a common closed system. The common closed system defines the chamber 514. The plunger 508 is arranged inside the closed system defined by the operating barrel 504 and the actuating barrel 506. The actuating barrel 506 may form a biasing member. In an example, the pneumatically actuated semi-automatically controllable syringe 500 includes a pneumatically controlled drive unit. The biasing member of the pneumatically actuated semi-automatically controllable syringe 500 may be the pneumatically controlled drive unit.

A slot is disposed on an external surface of one of the operating barrel 504 and the actuating barrel 506. The actuating member 510 is disposed on the external surface of one of the operating barrel 504 and the actuating barrel 506 and is coupled with the plunger 508 via the slot. The movement of the plunger 508 in direction A on application of a sliding force on the actuating member 510, creates a pneumatic pressure inside the actuating barrel 506. Upon removal of the sliding force, the movement of the plunger 508 to an original position towards direction B, is actuated with effect of the pneumatic pressure created inside the actuating barrel 506.

The movement control mechanism 516 includes a pin 516A and a set of pin-receiving members 516B. The set of pin-receiving members 516B are disposed on an external surface of the operating barrel 504 to control a range of displacement of the plunger 508.

The movement control mechanism 516 controls a range of displacement of the actuating member 510 by restricting the movement of the actuating member 510 to a required degree. Such controlling also limits a range of displacement of the plunger 508 due to the coupling of the actuating member 510 with the plunger 508. The pin 516A is inserted in one of the pin-receiving members 516B. The pin 516A protrudes out from the barrel 504 and abuts with the actuating member 510 upon the movement of the actuating member 510 in direction A. Such abutting limits further movement of the actuating member 510 in direction A. Further, due to the coupling of the actuating member 510 with the plunger 508, the movement of the plunger 508 is also limited in direction A. Similarly, by inserting the pin 516A in different pin-receiving members 516B a user may increase or decrease the range in accordance with the required operation.

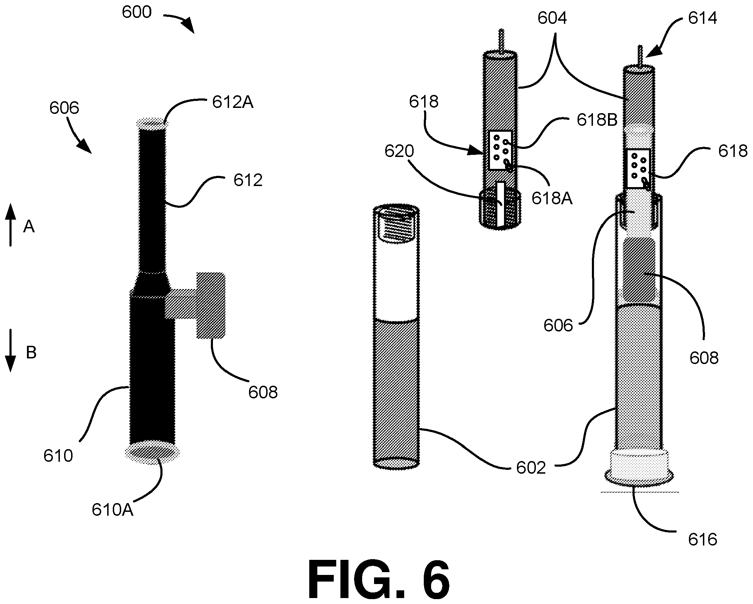

FIG. 6 illustrates a pneumatically actuated semi-automatically controllable syringe 600, in accordance with an implementation of the present subject matter. The pneumatically actuated semi-automatically controllable syringe 600 may be referred hereinafter as the pneumatic syringe 600, for the sake of brevity. The pneumatic syringe 600 includes a first tube 602, a second tube 604, a plunger 606, an actuating member 608, and a movement control mechanism 618. In an assembled state, the first tube 602 and a second tube 604 may form a barrel. The plunger 606 includes two cylindrical sections 610, 612. A first section 610 may have an inner diameter in a range of 5-15 mm. The outer edge of the first section 610 is provided with a sealing element 610A. A second section 612 may have a diameter in a range of 3-7 mm. Similar to the first section 610, an outer edge of the second section 612 is disposed with a sealing element 612A. In an example, the sealing elements 610A, 612A of both the sections 610, 612 of the plunger 606 may be made of a rubber voucher or gasket. The second tube 604 includes a slot 620. The actuating member 608 is disposed on the first tube 602 and coupled with the plunger 606 via the slot 620.

The plunger 606 is inserted inside the first tube 602 from a first end of the first tube 602. The second tube 604 having an outer diameter less than the inner diameter of the first tube 602, is attached to the first tube 602 from a second end of the first tube 602. The second tube 604 includes a protrusion 614 having a small opening. The protrusion 614 may have an inner diameter less than the inner diameter if the rest of the second tube 604. The outer diameter of the protrusion 614 may be in a range of 0.5-1.5 mm. The second tube 604 is attached to the second end of the first tube 602 in a manner to create a chamber. In an example, the first tube 602 is attached with the second tube 604 through a threaded connection. In another example, the first tube 602 is attached with the second tube 604 with a snap-fit connection in an air-tight manner.

In an arranged state, an operating end of the plunger 606, including a sealing element 612A, is arranged inside the second tube 604 and an actuating end of the plunger 606 is arranged inside the first tube 602. A cap 616 is attached to an end of the first tube 602, which is located away from the second tube 604, The cap 616 creates an air-tight enclosure inside the first tube 602, between the actuating end of the plunger 606 and the end of the first tube 602 on which the cap 616 is connected. In an example, the cap 114 is attached to the first tube 602 through a threaded connection. In another example, the cap 114 is snap-fitted onto the end of the first tube 602.

The movement control mechanism 618 controls a range of displacement of the actuating member 608 by restricting the movement of the actuating member 608 to a required degree. Such controlling also limits a range of displacement of the plunger 606 due to the coupling of the actuating member 608 with the plunger 606. The movement control mechanism 618 includes a pin 618A and a set of pin-receiving members 618B. The pin 618A is inserted in one of the pin-receiving members 618B. The pin 618A protrudes out from the second tube 604 and abuts with the actuating member 608 upon the movement of the actuating member 608 in direction A. Such abutting limits further movement of the actuating member 608 in direction A. Further, due to the coupling of the actuating member 608 with the plunger 606, the movement of the plunger 606 is also limited in direction A. Similarly, by inserting the pin 618A in different pin-receiving members 618B a user may increase or decrease the range in accordance with the required operation.

The plunger 606, arranged inside the first tube 602, is partially slidable towards the second tube 604 along the chamber. After assembling the first tube 602, the second tube 604, and the plunger 606 along with the actuating member 608, the cap 616 is used to close the first tube 602, from the end from where the plunger 606 is inserted into the first tube 602. The cap 616 creates an air-tight close system inside the first tube 602. The actuation of the actuating member 608 by application of a sliding force moves the plunger 606 in one direction while creating a negative pressure in a region opposite to the direction of the movement. This results in sliding back movement of the plunger 606 upon release of the applied force on the actuating member 608.

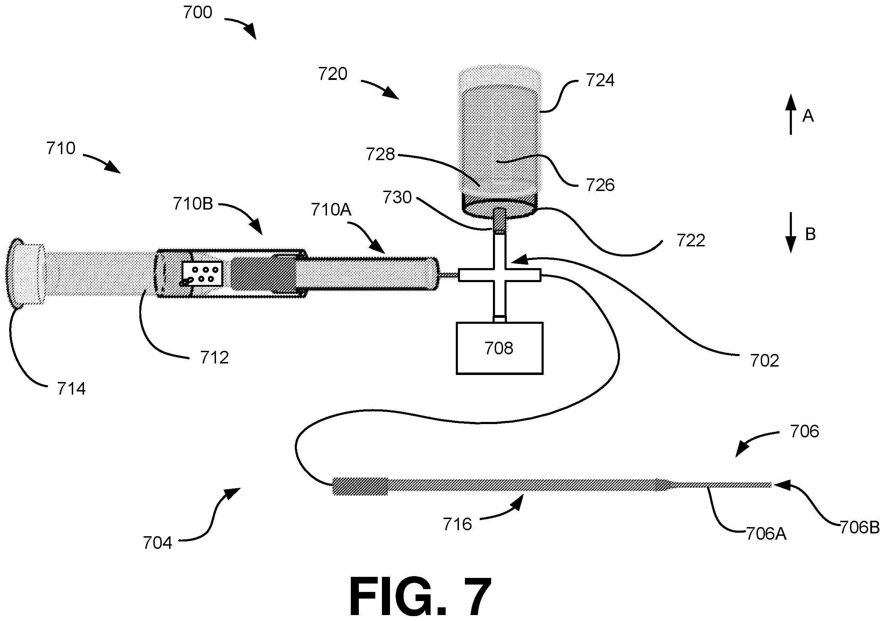

FIG. 7 illustrates a syringe assembly 700, in accordance with an implementation of the present subject matter. The syringe assembly 700 includes a coupler 702, a tube 704, a needle 706, a pressure sensor 708, at least one semi-automatically controllable syringe 710, and a manually controllable syringe 720. The manually controllable syringe 720 is a manually controllable rotary syringe 720. Although the manually controllable rotary syringe 720 is shown in FIG. 7, a manually controllable longitudinal syringe may be used in the syringe assembly 700. The at least one semi-automatically controllable syringe 710 is a longitudinal semi-automatically controllable syringe 710. Although the longitudinal semi-automatically controllable syringe 710 is shown in FIG. 7, a rotary semi-automatically controllable syringe may be used in the syringe assembly 700.

In an example, the longitudinal semi-automatically controllable syringe 710 is for creating an aspiration pressure required for oolemma puncturing and the manually controllable rotary syringe 720 is for sperm collection and sperm deposition upon successful puncturing of the oolemma. The longitudinal semi-automatically controllable syringe 710 of FIG. 7 may be similar to the semi-automatically controllable syringe 400 of FIG. 4. The longitudinal semi-automatically controllable syringe 710 includes a front cylindrical portion 710A and a rear cylindrical portion 710B. The rear cylindrical portion 710B accommodates a biasing member 712 and a cap 714 to limit the movement of the biasing member. The biasing member 712 and the cap 714 may be similar to the biasing member 410 and the cap 420 of FIG. 4. The front cylindrical portion 710A may have a diameter of 5-20 mm. The front cylindrical portion 710A is coupled to the coupler 702 via an adapter 716. The coupler 702, in turn, is coupled to the needle 706 via the tube 704. In an example, the needle 706 is mounted on a needle holder 718 for stably holding the needle 706. The needle holder 718 is mounted on a manipulator controller (not shown) in order to precisely control the positioning of the needle 706. The needle 706 includes a hollow shaft 706A with a tip 706B. An inner diameter of the tip 706B may be in a range of 20-50 microns. In an example, the needle 706 is a disposable needle.

The manually controllable rotary syringe 720 is also referred hereinafter as the rotary syringe 720 for the sake of brevity. The rotary syringe 720 includes a barrel 722, an actuating member 724, a threaded member 726, and a piston 728 coupled with the threaded member 726. In an example, the actuating member 724 is a rotary actuating member. The actuating member 724 is cylindrical in shape. The barrel 722 includes a protrusion 730 formed with a through opening (not shown). The through opening is coupled with the coupler 702 in order to couple with the needle 706 via the tube 704, and positioned at an end positioned opposite to the piston 728. A movement of the piston 728 inside the barrel 722 may be in a range of 1-3 cm. An inner diameter of the barrel 722 may be in a range of 20-40 mm. The actuating member 724 includes a body with threaded inner surface and the threaded member 726 have threads which complement with the threads of the body. The threads of the threaded member 726 engages with the threads of the body. Further, the body is coupled with the longitudinal semi-automatically controllable syringe 710 via the coupler 702.

The coupler 702 is connected to the pressure sensor 708 and the tube 704 connected to the needle 706. The length of the tube 704 may be in a range of 70-200 cm. The pressure sensor 708 is utilized to measure the value of pressure being applied from the longitudinal semi-automatically controllable syringe 710 and the rotary syringe 720. The coupler 702 provides a junction between the longitudinal semi-automatically controllable syringe 710, the rotary syringe 720, the pressure sensor 708, and the needle 706. An inner diameter of the tube 704 may be 1 mm. A clockwise movement of the actuating member 724 causes a movement of the piston 728 in direction A due to the engaging of the threads of the threaded member 726 and the threads of the body. The movement of the piston 728 in direction A may create a negative pressure inside the barrel 722. Alternatively, a movement of the actuating member 724 in an anti-clockwise direction causes a movement of the piston 728 in direction B due to the engaging of the threads of the threaded member 726 and the threads of the body. The movement of the piston 728 in direction B creates a positive pressure inside the barrel 722.

The creation of the negative pressure in the rotary syringe 720 is performed after placing the needle 706 in close proximity to a to-be-injected sperm, which allows collection of the sperm into the tube 704 connected to the rotary syringe 720. The tip 706B is placed in contact with a target oocyte (not shown). An outer layer (not shown) of the oocyte is pierced with a mechanical force applied on the outer layer through the needle 706. After piercing the outer layer further mechanical force is applied to move the tip 706B into a range of 30% to 70% of the diameter of the oocyte. The longitudinal semi-automatically controllable syringe 710 is used to puncture an inner layer (oolemma) of the oocyte through the needle 706 as explained in detail in FIG. 4. After successful puncturing of the oolemma, in order to perform deposition of the collected sperm in the oocyte, the actuating member 724 of the rotary syringe 720 is rotated in the anti-clockwise direction, which creates a positive pressure in the rotary syringe 720. The positive pressure results in the ejection of the sperm towards an opening of the needle 706. The needle 706 is placed in contact with the target region of the oocyte, and further positive pressure is applied by the rotary syringe 720 in order to deposit the sperm into the oocyte.

Operating the syringe assembly 700 as recited in the implementation of the present subject matter, allows for performing the sperm injection process without damaging the oocyte in a precise manner by removing the effect of subjectivity involved in the oolemma puncturing process. In addition, and semi-automation of the oolemma puncturing process allows for reducing the overall time required for performing the intracytoplasmic sperm injection (ICSI) process with required pressure applied onto the oocyte, reduces the time required for oocyte manipulation and also minimizes the subjectivity of the user for ICSI procedure performance.

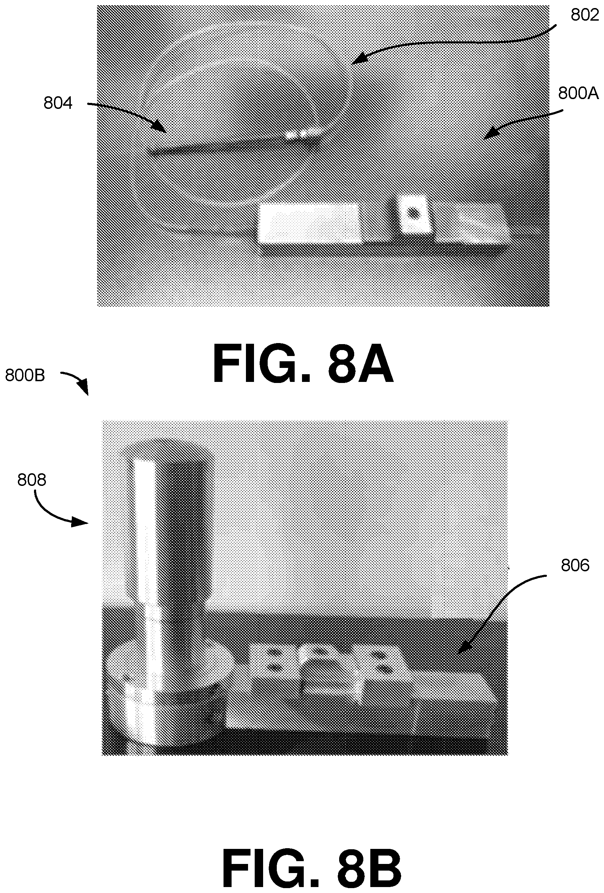

FIG. 8A illustrates a longitudinal semi-automatically controllable syringe 800A, in accordance with an implementation of the present subject matter. The longitudinal semi-automatically controllable syringe 800A is pneumatically actuated. The longitudinal semi-automatically controllable syringe 800A may be similar to the pneumatically actuated semi-automatically controllable syringe 500 of FIG. 5A, and may be used for holding an oocyte for performing sperm injection. The longitudinal semi-automatically controllable syringe 800A is connected to a tube 802 and a needle 804 for performing the process of oocyte holding as explained in detail in FIGS. 2 & 3. Length of the tube is in a range of 100-150 cm. An outer diameter of a tip of the needle 804 may be in a range of 60-100 micro-meters, and an inner diameter of the tube 802 is in a range of 10-30 microns.

FIG. 8B illustrates an injection assembly 800B, in accordance with an implementation of the present subject matter. The injection assembly 800B includes a longitudinal semi-automatically controllable syringe 806 and a manually controllable rotary syringe 808 is similar to the longitudinal semi-automatically controllable syringe 710 and the rotary syringe 720 of FIG. 7. The injection assembly is connected to an injection side tube (not shown) and an injection side needle (not shown) for performing the process of sperm injection. The injection side needle have an inner diameter in a range of 5-10 micro-meters. The injection side tube have a length in a range of 100-200 cm.

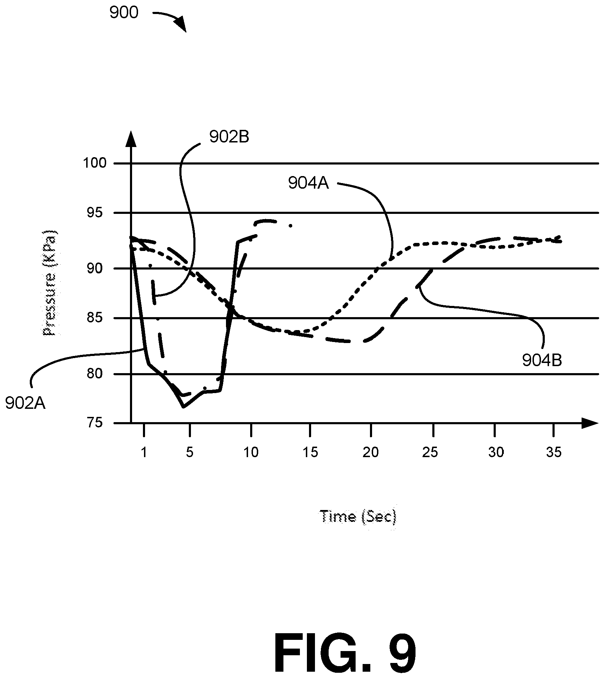

FIG. 9 illustrates a graphical representation 900 of the performance comparison between a conventional syringe and an exemplary semi-automatically controllable syringe with respect to data points representing pressure values measured at an injection needle side for an intracytoplasmic sperm injection process. Graph of FIG. 9 represents the comparison of data representing time (in seconds) on the x-axis time and pressure scale (in kilo-pascal) on the y-axis pertaining to conventional intracytoplasmic sperm injection (ICSI) technique and exemplary technique of the present subject matter on discarded oocytes. Referring to FIG. 9, 902A and 902B represents data points of readings pertaining to the exemplary semi-automatically controllable syringe and 904A and 904B represents data points of readings pertaining to the conventional syringe. The graphical representation 900 illustrates a drastic difference between the time taken to perform intracytoplasmic sperm injection process with respect to the conventional syringe and the exemplary semi-automatically controllable syringe.

## Claims

1. A semi-automatically controllable syringe (100A-100E, 200, 300, 400, 500, 600, 710, 800A) comprising:
a barrel (102A, 102B, 202, 302, 402) including an orifice and an internal cavity;
a plunger (104, 204, 304, 404, 508, 606) including an actuating end and an operating end, wherein the plunger (104, 204, 304, 404, 508, 606) is at least partially disposed in the internal cavity of the barrel (102A, 102B, 202, 302, 402, 504) and is slidable in a first direction and a second direction opposite to the first direction in the internal cavity, and wherein the operating end of the plunger (104, 204, 304, 404, 508, 606) and the orifice of the barrel (102A, 102B, 202, 302, 402, 504) defines a chamber (112, 212, 312, 412, 514);
an actuating member (116, 124, 206, 306, 406, 510, 608) coupled with the plunger (104, 204, 304, 404, 508, 608), wherein the actuating member (116, 206, 306, 406, 510, 608), upon application of an actuating force thereon, is to cause a movement of the plunger (104, 204, 304, 404, 508, 606) in the first direction;
a biasing member (110, 210, 310, 410, 506) coupled with the plunger (104, 204, 304, 404, 508, 606), wherein the biasing member (110, 210, 310, 410, 506) is to bias upon movement of the plunger (104, 204, 304, 404, 508, 606) in the first direction and is to apply a restoring force on the plunger (104, 204, 304, 404, 508, 606) towards the second direction;
**characterized in that** it also comprises
a movement control mechanism (118, 120, 218, 314, 418, 516, 618) including a pin (118A, 120A, 218A, 314A, 418A, 516A, 618A) and a set of pin-receiving members (118B, 120B, 218B, 314B, 418B, 516B, 618B), wherein the pin (118A, 120A, 218A, 314A, 418A, 516A, 618A) is insertable in any of the pin-receiving members (118B, 120B, 218B, 314B, 418B, 516B, 618B), from the set of pin-receiving members (118B, 120B, 218B, 314B, 418B, 516B, 618B), to provide a variable limit for the movement of the plunger (104, 204, 304, 404, 508, 606) within the barrel (102A, 102B, 202, 302, 402, 504), in the first direction.

2. The semi-automatically controllable syringe (100A, 200, 300, 400, 500, 600, 710, 800A) as claimed in claim 1, wherein the set of pin-receiving members (118B, 218B, 314B, 418B, 516B, 618B) are on an outer surface of the barrel (102A, 202, 302, 402, 504).

3. The semi-automatically controllable syringe (100B-100E) as claimed in claim 1, wherein the movement control mechanism (120) comprises a sleeve (122) mountable on the barrel (102B), and wherein the set of pin-receiving members (120B) are on the sleeve (122).

4. The semi-automatically controllable syringe (100A-100C, 200, 300, 400, 500, 600, 710, 800A) as claimed in claim 1, wherein the set of pin-receiving members (118B, 120B, 218B, 314B, 418B, 516B, 618B) are aligned along a straight line inclined with respect to a longitudinal axis of the semi-automatically controllable syringe (100A-100C, 200, 300, 400, 500, 600, 710, 800A).

5. The semi-automatically controllable syringe (100D and 100E) as claimed in claim 1, wherein the set of pin-receiving members (120B) are aligned on a zig-zag line.

6. The semi-automatically controllable syringe (100A-100E, 200, 300, 400, 500, 600, 710, 800A) as claimed in claim 1, wherein the set of pin-receiving members (118B, 120B, 218B, 314B, 418B, 516B, 618B) is a set of holes.

7. The semi-automatically controllable syringe (100A-100E, 200, 300, 400, 500, 600, 710, 800A) as claimed in claim 1, wherein the set of pin-receiving members (118B, 120B, 218B, 314B, 418B, 516B, 618B) is a set of cavities.

8. The semi-automatically controllable syringe (100A-100E, 200, 300, 400, 500, 600, 710, 800A) as claimed in claim 1, wherein the biasing member (110, 210, 310, 410) is a spring.

9. The semi-automatically controllable syringe (100A-100E, 200, 300, 400, 500, 600, 710, 800A) as claimed in claim 1, wherein the biasing member (506) is a pneumatically controlled drive unit.

10. The semi-automatically controllable syringe (100A-100E, 200, 300, 400, 500, 600, 710, 800A) as claimed in claim 1, wherein the actuating member (116, 124, 206, 306, 406, 510, 608) is one of a linear actuating member (116, 124, 206, 306, 406, 510, 608) and a rotary actuating member.

11. The semi-automatically controllable syringe (100A-100E, 200, 300, 400, 500, 600, 710, 800A) as claimed in claim 1, wherein the actuating member (116, 124, 206, 306, 406, 510, 608) is one of a hydraulic actuating member, a pneumatic actuating member, a mechanical actuating member, and an electrical actuating member.

12. A syringe assembly (700, 800B) comprising:
at least one semi-automatically controllable syringe (100A-100E, 200, 300, 400, 500, 600, 710, 800A) as claimed in one of claims 1 to 11;
a manually controllable syringe (720) including a barrel (722), an actuating member (724), a piston (728), and an orifice;
a coupler (702) connected to the orifice of the at least one semi-automatically controllable syringes (100A-100E, 200, 300, 400, 500, 600, 710, 800A) and the orifice of the manually controllable syringe (720);
a tube (704) having a first end coupled with the coupler (702) and a second end coupled with a needle (706); and
a pressure sensor (708) coupled with the coupler (702), wherein the pressure sensor (708) is to measure a value of pressure being applied from at least one of the at least one semi-automatically controllable syringes (100A-100E, 200, 300, 400, 500, 600, 710, 720, 800A) and the manually controllable syringe (720).

## Patentansprüche

1. Halbautomatisch steuerbare Spritze (100A-100E, 200, 300, 400, 500, 600, 710, 800A), umfassend:
einen Zylinder (102A, 102B, 202, 302, 402) mit einer Öffnung und einem inneren Hohlraum;
einen Kolben (104, 204, 304, 404, 508, 606), der ein Betätigungsende und ein Arbeitsende aufweist, wobei der Kolben (104, 204, 304, 404, 508, 606) zumindest teilweise in dem inneren Hohlraum des Zylinders (102A, 102B, 202, 302, 402, 504) angeordnet ist und in einer ersten Richtung und einer zweiten Richtung entgegengesetzt zu der ersten Richtung in dem inneren Hohlraum verschiebbar ist, und wobei das Arbeitsende des Kolbens (104, 204, 304, 404, 508, 606) und die Öffnung des Zylinders (102A, 102B, 202, 302, 402, 504) eine Kammer (112, 212, 312, 412, 514) definieren;
ein Betätigungselement (116, 124, 206, 306, 406, 510, 608), das mit dem Kolben (104, 204, 304, 404, 508, 608) gekoppelt ist, wobei das Betätigungselement (116, 206, 306, 406, 510, 608) beim Aufbringen einer Betätigungskraft darauf eine Bewegung des Kolbens (104, 204, 304, 404, 508, 606) in der ersten Richtung bewirkt;
ein Vorspannelement (110, 210, 310, 410, 506), das mit dem Kolben (104, 204, 304, 404, 508, 606) gekoppelt ist, wobei das Vorspannelement (110, 210, 310, 410, 506) dazu dient, bei der Bewegung des Kolbens (104, 204, 304, 404, 508, 606) in die erste Richtung vorzuspannen und eine Rückstellkraft auf den Kolben (104, 204, 304, 404, 508, 606) in die zweite Richtung auszuüben;
**dadurch gekennzeichnet, dass** sie außerdem umfasst
einen Bewegungssteuerungsmechanismus (118, 120, 218, 314, 418, 516, 618) mit einem Stift (118A, 120A, 218A, 314A, 418A, 516A, 618A) und einem Satz von Stiftaufnahmeelementen (118B, 120B, 218B, 314B, 418B, 516B, 618B), wobei der Stift (118A, 120A, 218A, 314A, 418A, 516A, 618A) in ein beliebiges Stiftaufnahmeelement (118B, 120B, 218B, 314B, 418B, 516B, 618B) aus dem Satz von Stiftaufnahmeelementen (118B, 120B, 218B, 314B, 516B, 618B) einführbar ist, um eine variable Grenze für die Bewegung des Kolbens (104, 204, 304, 404, 508, 606) innerhalb des Zylinders (102A, 102B, 202, 302, 402, 504) in der ersten Richtung zu schaffen.

2. Halbautomatisch steuerbare Spritze (100A, 200, 300, 400, 500, 600, 710, 800A) nach Anspruch 1, wobei sich der Satz von Stiftaufnahmeelementen (118B, 218B, 314B, 418B, 516B, 618B) auf einer Außenfläche des Zylinders (102A, 202, 302, 402, 504) befindet.

3. Halbautomatisch steuerbare Spritze (100B-100E) nach Anspruch 1, wobei der Bewegungssteuerungsmechanismus (120) eine Hülse (122) umfasst, die auf dem Zylinder (102B) montiert werden kann, und wobei sich der Satz von Stiftaufnahmeelementen (120B) auf der Hülse (122) befindet.

4. Halbautomatisch steuerbare Spritze (100A-100C, 200, 300, 400, 500, 600, 710, 800A) nach Anspruch 1, wobei der Satz von Stiftaufnahmeelementen (118B, 120B, 218B, 314B, 418B, 516B, 618B) entlang einer geraden Linie ausgerichtet ist, die in Bezug auf eine Längsachse der halbautomatisch steuerbaren Spritze (100A-100C, 200, 300, 400, 500, 600, 710, 800A) geneigt ist.

5. Halbautomatisch steuerbare Spritze (100D und 100E) nach Anspruch 1, wobei der Satz von Stiftaufnahmeelementen (120B) auf einer Zickzacklinie ausgerichtet ist.

6. Halbautomatisch steuerbare Spritze (100A-100E, 200, 300, 400, 500, 600, 710, 800A) nach Anspruch 1, wobei der Satz von Stiftaufnahmeelementen (118B, 120B, 218B, 314B, 418B, 516B, 618B) ein Satz von Löchern ist.

7. Halbautomatisch steuerbare Spritze (100A-100E, 200, 300, 400, 500, 600, 710, 800A) nach Anspruch 1, wobei der Satz von Stiftaufnahmeelementen (118B, 120B, 218B, 314B, 418B, 516B, 618B) ein Satz von Hohlräumen ist.

8. Halbautomatisch steuerbare Spritze (100A-100E, 200, 300, 400, 500, 600, 710, 800A) nach Anspruch 1, wobei das Vorspannelement (110, 210, 310, 410) eine Feder ist.

9. Halbautomatisch steuerbare Spritze (100A-100E, 200, 300, 400, 500, 600, 710, 800A) nach Anspruch 1, wobei das Vorspannelement (506) eine pneumatisch gesteuerte Antriebseinheit ist.

10. Halbautomatisch steuerbare Spritze (100A-100E, 200, 300, 400, 500, 600, 710, 800A) nach Anspruch 1, wobei das Betätigungselement (116, 124, 206, 306, 406, 510, 608) eines von einem linearen Betätigungselement (116, 206, 306, 406, 510, 608) und einem drehbaren Betätigungselement ist.

11. Halbautomatisch steuerbare Spritze (100A-100E, 200, 300, 400, 500, 600, 710, 800A) nach Anspruch 1, wobei das Betätigungselement (116, 124, 206, 306, 406, 510, 608) eines von einem hydraulischen Betätigungselement, einem pneumatischen Betätigungselement, einem mechanischen Betätigungselement und einem elektrischen Betätigungselement ist.

12. Spritzenanordnung (700, 800B), umfassend:
mindestens eine halbautomatisch steuerbare Spritze (100A-100E, 200, 300, 400, 500, 600, 710, 800A) nach einem der Ansprüche 1 bis 11;
eine manuell steuerbare Spritze (720) mit einem Zylinder (722), einem Betätigungselement (724), einem Kolben (728) und einer Öffnung;
einen Koppler (702), der mit der Öffnung der mindestens eine halbautomatisch steuerbare Spritze (100A-100E, 200, 300, 400, 500, 600, 710, 800A) und mit der Öffnung der manuell steuerbaren Spritze (720) verbunden ist;
ein Rohr (704) mit einem ersten Ende, das mit dem Koppler (702) verbunden ist, und einem zweiten Ende, das mit einer Nadel (706) verbunden ist; und
einen Drucksensor (708), der mit dem Koppler (702) gekoppelt ist, wobei der Drucksensor (708) dazu dient, einen Wert des Drucks zu messen, der von mindestens einer der halbautomatisch steuerbaren Spritzen (100A-100E, 200, 300, 400, 500, 600, 710, 720, 800A) und der manuell steuerbaren Spritze (720) angelegt wird.

## Revendications

1. Seringue à commande semi-automatique (100A-100E, 200, 300, 400, 500, 600, 710, 800A) comprenant :
un fût (102A, 102B, 202, 302, 402) comprenant un orifice et une cavité interne ;
un piston (104, 204, 304, 404, 508, 606) comprenant une extrémité d'actionnement et une extrémité de fonctionnement, dans lequel le piston (104, 204, 304, 404, 508, 606) est au moins partiellement disposé dans la cavité interne du fût (102A, 102B, 202, 302, 402, 504) et peut coulisser dans une première direction et une seconde direction opposée à la première direction dans la cavité interne, et dans lequel l'extrémité de fonctionnement du piston (104, 204, 304, 404, 508, 606) et l'orifice du fût (102A, 102B, 202, 302, 402, 504) définissent une chambre (112, 212, 312, 412, 514) ;
un élément d'actionnement (116, 124, 206, 306, 406, 510, 608) couplé au piston (104, 204, 304, 404, 508, 608), dans lequel l'élément d'actionnement (116, 206, 306, 406, 510, 608), lors de l'application d'une force d'actionnement sur celui-ci, provoque un mouvement du piston (104, 204, 304, 404, 508, 606) dans la première direction ;
un élément de sollicitation (110, 210, 310, 410, 506) couplé au piston (104, 204, 304, 404, 508, 606), dans lequel l'élément de sollicitation (110, 210, 310, 410, 506) exerce une pression sur le piston (104, 204, 304, 404, 508, 606) lors de son déplacement dans la première direction et applique une force de rappel sur le piston (104, 204, 304, 404, 508, 606) dans la seconde direction ;
**caractérisée en ce qu'**elle comprend également
un mécanisme de contrôle du mouvement (118, 120, 218, 314, 418, 516, 618) comprenant une goupille (118A, 120A, 218A, 314A, 418A, 516A, 618A) et un ensemble d'éléments récepteurs de goupille (118B, 120B, 218B, 314B, 418B, 516B, 618B), dans lequel la goupille (118A, 120A, 218A, 314A, 418A, 516A, 618A) peut être insérée dans n'importe quel élément récepteur de goupille (118B, 120B, 218B, 314B, 418B, 516B, 618B) de l'ensemble d'éléments récepteurs de goupille (118B, 120B, 218B, 314B, 516B, 618B), afin de fournir une limite variable pour le mouvement du piston (104, 204, 304, 404, 508, 606) à l'intérieur du fût (102A, 102B, 202, 302, 402, 504), dans la première direction.

2. Seringue à commande semi-automatique (100A, 200, 300, 400, 500, 600, 710, 800A) selon la revendication 1, dans laquelle l'ensemble des éléments récepteurs de goupilles (118B, 218B, 314B, 418B, 516B, 618B) se trouve sur une surface extérieure du corps (102A, 202, 302, 402, 504).

3. Seringue à commande semi-automatique (100B-100E) selon la revendication 1, dans laquelle le mécanisme de contrôle du mouvement (120) comprend un manchon (122) pouvant être monté sur le fût (102B), et dans laquelle l'ensemble des éléments récepteurs de goupilles (1206) se trouve sur le manchon (122).

4. Seringue à commande semi-automatique (100A-100C, 200, 300, 400, 500, 600, 710, 800A) selon la revendication 1, dans laquelle l'ensemble des éléments récepteurs de goupilles (118B, 120B, 218B, 314B, 418B, 516B, 618B) sont alignés le long d'une ligne droite inclinée par rapport à un axe longitudinal de la seringue à commande semi-automatique (100A-100C, 200, 300, 400, 500, 600, 710, 800A).

5. Seringue à commande semi-automatique (100D et 100E) selon la revendication 1, dans laquelle l'ensemble des éléments récepteurs de goupilles (1206) sont alignés sur une ligne en zig-zag.

6. Seringue à commande semi-automatique (100A-100E, 200, 300, 400, 500, 600, 710, 800A) selon la revendication 1, dans laquelle l'ensemble des éléments récepteurs de goupilles (118B, 120B, 218B, 314B, 418B, 516B, 618B) est un ensemble de trous.

7. Seringue à commande semi-automatique (100A-100E, 200, 300, 400, 500, 600, 710, 800A) selon la revendication 1, dans laquelle l'ensemble des éléments récepteurs de goupilles (118B, 120B, 218B, 314B, 418B, 516B, 618B) est un ensemble de cavités.

8. Seringue à commande semi-automatique (100A-100E, 200, 300, 400, 500, 600, 710, 800A) selon la revendication 1, dans laquelle l'élément de sollicitation (110, 210, 310, 410) est un ressort.

9. Seringue à commande semi-automatique (100A-100E, 200, 300, 400, 500, 600, 710, 800A) selon la revendication 1, dans laquelle l'élément de sollicitation (506) est une unité d'entraînement à commande pneumatique.

10. Seringue à commande semi-automatique (100A-100E, 200, 300, 400, 500, 600, 710, 800A) selon la revendication 1, dans laquelle l'élément d'actionnement (116, 124, 206, 306, 406, 510, 608) est l'un parmi un élément d'actionnement linéaire (116, 124, 206, 306, 406, 510, 608) et un élément d'actionnement rotatif.

11. Seringue à commande semi-automatique (100A-100E, 200, 300, 400, 500, 600, 710, 800A) selon la revendication 1, dans laquelle l'organe d'actionnement (116, 124, 206, 306, 406, 510, 608) est l'un parmi un organe d'actionnement hydraulique, un organe d'actionnement pneumatique, un organe d'actionnement mécanique et un organe d'actionnement électrique.

12. Ensemble de seringue (700, 800B) comprenant :
au moins une seringue à commande semi-automatique (100A-100E, 200, 300, 400, 500, 600, 710, 800A) selon l'une des revendications 1 à 11 ;
une seringue à commande manuelle (720) comprenant un fût (722), un élément d'actionnement (724), un piston (728) et un orifice ;
un coupleur (702) connecté à l'orifice d'au moins une seringue à contrôle semi-automatique (100A-100E, 200, 300, 400, 500, 600, 710, 800A) et à l'orifice de la seringue à contrôle manuel (720) ;
un tube (704) ayant une première extrémité couplée au coupleur (702) et une seconde extrémité couplée à une aiguille (706) ; et
un capteur de pression (708) couplé au coupleur (702), dans lequel le capteur de pression (708) mesure une valeur de pression appliquée par au moins une des seringues à commande semi-automatique (100A-100E, 200, 300, 400, 500, 600, 710, 720, 800A) et la seringue à commande manuelle (720).
